Europäisches Patentamt

European Patent Office

Office européen des brevets

(19)

(11) **EP 1 161 456 B1**

(12) **EUROPÄISCHE PATENTSCHRIFT**

(45) Veröffentlichungstag und Bekanntmachung des Hinweises auf die Patenterteilung:
**29.12.2004 Patentblatt 2004/53**

(21) Anmeldenummer: **00920490.0**

(22) Anmeldetag: **10.03.2000**

(51) Int Cl.[7]: **C07K 16/28**, C07K 16/46, A61K 39/395, A61K 38/19, A61K 47/48, A61P 37/00, C12N 5/10, C12N 1/21

(86) Internationale Anmeldenummer:
**PCT/EP2000/002154**

(87) Internationale Veröffentlichungsnummer:
**WO 2000/053633 (14.09.2000 Gazette 2000/37)**

(54) **GEGEN CCR5 GERICHTETE ANTIKÖRPERKONSTRUKTE UND IHRE VERWENDUNG IN DER BEHANDLUNG VON AUTOIMMUNKRANKHEITEN**

CCR5 BINDING ANTIBODY CONSTRUCTS AND THEIR USE FOR TREATING AUTOIMMUNE DISEASES

CONSTRUCTIONS D'ANTICORPS SE LIANT AU CCR5 ET LEUR UTILISATION POUR LE TRAITEMENT DES MALADIES AUTO-IMMUNES

(84) Benannte Vertragsstaaten:
**AT BE CH CY DE DK ES FI FR GB GR IE IT LI LU MC NL PT SE**
Benannte Erstreckungsstaaten:
**LT LV SI**

(30) Priorität: **11.03.1999 DE 19910891**

(43) Veröffentlichungstag der Anmeldung:
**12.12.2001 Patentblatt 2001/50**

(73) Patentinhaber: **Micromet AG**
**81477 München (DE)**

(72) Erfinder:
• **MACK, Matthias**
**93051 Regensburg (DE)**
• **SCHLÖNDORFF, Detlef**
**80803 München (DE)**

(74) Vertreter: **VOSSIUS & PARTNER**
**Siebertstrasse 4**
**81675 München (DE)**

(56) Entgegenhaltungen:
WO-A-00/04926     WO-A-98/08957
WO-A-98/18826     WO-A-99/01127

• **BRUEHL HILKE ET AL: "High accumulation of leukocytes expressing the chemokine receptor CCR5 in inflammatory joint effusions." ARTHRITIS & RHEUMATISM, Bd. 41, Nr. 9 SUPPL., 1998, Seite S195 XP000916499 62nd National Scientific Meeting of the American College of Rheumatology and the 33rd National Scientific Meeting of the Association of Rheumatology Health Professionals;San Diego, California, USA; November 8-12, 1998 ISSN: 0004-3591**
• **MACK M ET AL: "A small bispecific antibody construct expressed as a functional single-chain molecule with high tumor cell cytotoxicity." PROCEEDINGS OF THE NATIONAL ACADEMY OF SCIENCES OF THE UNITED STATES OF AMERICA, (1995 JUL 18) 92 (15) 7021-5. , XP000566333**
• **BIRD P.I.: 'Regulation of pro-apoptotic leucocyte granule serine proteinases by intracellular serpins', IMMUNOLOGY AND CELL BIOLOGY, 1999, 77, 47-57**
• **SHRESTA ET AL: "How do cytotoxic lymphocytes kill their targets?" CURRENT OPINION IN IMMUNOLOGY, 1998, 10, 581-587**
• **SUN J. ET AL: 'A Cytosolic Granzyme B Inhibitor Related to the Viral Apoptotic Regulator Cytokine Response Modifier A is Present in Cytotoxic Lymphocytes', JOURNAL OF BIOLOGICAL CHEMISTRY, 1996, 271(44), 27802-27809**

**(Forts. nächste Seite)**

- BIRD C.H.: 'Selective Regaulation of Apoptosis: the Cytotoxic Lymphocyte Serpin Proteinase Inhibitor 9 Protects against Granzyme B-Mediated Apoptosis without Perturbing the Fas Cell Death Pathway', MOLECULAR AND CELLULAR BIOLOGY, 1998, 18(11), 6387-6398

**Beschreibung**

Die vorliegende Erfindung betrifft Antikörper konstrukte gegen Chemokinrezeptor exprimierende Zellen, insbesondere gegen den Chemokinrezeptor CCR5 exprimierende Monozyten/Makrophagen und CCR5[+] T-Zellen. Weiter betrifft die Erfindung die Verwendung von Antikörper konstrukten zur Zerstörung von Chemokinrezeptor exprimierenden Zellen zur Behandlung von Autoimmunkrankheiten und allergischen Erkrankungen, insbesondere zur Behandlung von chronisch entzündlichen Gelenkserkrankungen. Die erfindungsgemäßen Antikörper konstrukte ermöglichen die gezielte Depletion von Chemokinrezptoren exprimierenden Zellen und somit eine gezielte immunsuppressive Therapie von Autoimmunkrankheiten.

In Deutschland leiden ca. 1% der Bevölkerung an Rheumatoider Arthritis. Hinzu kommen noch eine Reihe weiterer Erkrankungen aus dem rheumatischen Formenkreis, die ebenfalls zu Gelenksentzündungen führen. Für die Behandlung entzündlicher Gelenkserkrankungen werden gegenwärtig drei Gruppen von Medikamenten eingesetzt: Nichtsteroidale Antirheumatika, Kortisonpräparate, Basistherapeutika und TNFα-Blokkade. Bisheriger Therapieschwerpunkt ist die lokale Injektion von Kortisonpräparaten zusammen mit einer systemischen Gabe von Antiphlogistika oder Basistherapeutika.

Nichtsteroidale Antirheumatika entfalten eine milde schmerzlindernde und entzündungs-hemmende Wirkung, zeichnen sich aber bei häufiger Anwendung durch eine hohe Nebenwirkungsrate aus (z.B. Magengeschwüre, Nierenschädigungen). Kortisonpräparate wirken in hoher Dosierung stark abschwellend und schmerzlindernd, wobei es jedoch nach Absetzen zu einem schnellen Rezidiv kommt Zudem können Kortisonpräparate den Zerstörungsprozeß der Gelenkserkrankung nicht aufhalten. Eine langfristige Therapie mit Kortison ist in der Regel mit schwerwiegenden Nebenwirkungen verbunden (Infektionen, Cushing-Phänomen, Osteoporose, Pergamenthaut, Stoffwechsel- und Hormonstörungen). Auch die lokale Kortisoninjektion hat den wesentlichen Nachteil, daß die eingewanderten weißen Blutkörperchen nur in ihrer Aktivität gedämpft und nicht zerstört sind, weshalb es nach Absetzen der Behandlung zu einem schnellen Rezidiv kommt, was wie oben erwähnt auch für die systemische Anwendung gilt. Selten tritt nach der Kortisoninjektion ein Aufflammen der Entzündung aufgrund der irritierenden Wirkung von Kortisonkristallen auf. Die Wirkungsdauer einer Kortisoninjektion ist sehr variabel und reicht von primärer Wirkungslosigkeit bis zu einer Wirkung für mehrere Wochen.

Basistherapeutika werden in der Rheumatologie angewandt, um eine langfristige Unterdrückung der Entzündung und Einsparung von Kortisonpräparaten zu bewirken. Aufgrund erheblicher Toxizität (Allergien, Infekte, maligne Erkrankungen, Nierenfunktionsverschlechterung, Blutdruckkrisen, Lungenerkrankungen) ist eine engmaschige fachärztliche Betreuung der Patienten notwendig. Mit einem Wirkungseintritt ist nicht vor 3 Monaten nach Beginn der Behandlung zu rechnen. Zur Verfügung stehen derzeit 4 bis 5 verschiedene Basistherapeutika, die zunächst einzeln eingesetzt oder bei Therapieversagen kombiniert werden. Über den Wirkungsmechanismus der Basistherapeutika ist meist nur wenig bekannt. Ob eine Gelenksdestruktion durch Anwendung von Basistherapeutika vermindert werden kann, ist nicht endgültig geklärt. In den letzten Jahren wurde eine neue Substanzgruppe in die Therapie der rheumatoiden Arthritis eingeführt, die auf der Blockade von Zellbotenstoffen (insbesondere TNFα) mittels monoklonaler Antikörper oder löslicher Rezeptorkonstrukte beruht.

Darüber hinaus sprechen immer wieder Patienten nicht auf die derzeit verfügbaren Therapien an; in anderen Fällen muß die herkömmliche Behandlung aufgrund von intolerablen Nebenwirkungen abgebrochen werden.

WO 00/04926 beschreibt Chemokinrezeptorkonjugate, die aus Chemokinrezeptor bindenden Agenzien und Toxinen bestehen. Diese Konjugate werden zur Behandlung von entzündlichen Antworten eingesetzt.

WO 98/18826 offenbart monoklonale Antikörper mit Bindespezifität für Säuger-CCR5 und beschreibt die antagonistische Inhibition der CCR5-Ligandenbindung.

WO 98/08957 offenbart chimäre Konstrukte, die spezifisch an IL-13 Rezeptoren binden können. Die entsprechenden chimären Moleküle- sollen, zur Behandlung von Tumoren eingesetzt werden.

Bruehl (1998) Arthritis & Rheumatism (41; Suppl.) S.195 beschreibt, dass CCR5-positive Zellen in der Synovialflüssigkeit von Patienten mit Arthritis, gefunden werden können. Eine entsprechende Akkumulation der CCR5-positiven Zellen kann für die Pathogenese oder das Anhalten einer entzündlichen Reaktion wichtig sein.

Es besteht somit ein Bedürfnis für ein neues therapeutisches Prinzip für die Behandlung chronisch entzündlicher Gelenkserkrankungen bzw. von rheumatischen Erkrankungen allgemein und anderen Autoimmunerkrankungen.

Aufgabe der vorliegenden Erfindung ist es demnach, neue therapeutische Wirkstoffe zur Behandlung von entzündlichen Gelenkserkrankungen und anderen Autoimmunkrankheiten sowie allergischen Erkrankungen bereitzustellen, die die Nachteile des Standes der Technik überwinden.

Zur Lösung dieser Aufgabe dienen die Merkmale der unabhängigen Schutzansprüche.

Vorteilhafte Ausgestaltungen sind in den jeweiligen Unteransprüchen definiert.

Die Aufgabe wird gelöst durch Antikörper konstrukte, die in der Lage sind, an einen Chemokinrezeptor auf der Oberfläche einer Targetzelle zu binden, wobei die Bindung des Konstrukts an den Chemokinrezeptor in der Zerstörung der Targetzelle resultiert. Die erfindungsgemäßen Antikörper konstrukte bewirken eine selektive Zerstörung von Chemokinrezeptor exprimierenden Targetzellen, insbesondere Leukozyten. Die erfindungsgemäßen Konstrukte umfassen bispezifische Antikörper und binden sowohl an eine Chemokinrezeptor exprimierende Targetzelle als auch an ein CD3-Antigen auf der Oberfläche von Effektorzellen. Bei den Effektorzellen handelt es sich vorzugsweise um Leukozyten, insbesondere Monozyten/Makrophagen oder T-Zellen, oder dendritische Zellen.

In einer Studie an über 40 Patienten mit verschiedenen Gelenkerkrankungen wurde gefunden, daß es im entzündeten Gelenk zu einer sehr starken Anreichung von den Chemokin-rezeptor CCR5 exprimierenden Monozyten/Makrophagen und T-Lymphozyten kommt. Im Gelenk exprimieren über 90% der Monozyten und T-Lymphozyten den Chemokinrezeptor CCR5, während dies im Blut nur etwa 10% bzw. 20% sind. Aufgrund dieses Expressionsmusters sind der Chemokinrezeptor CCR5 bzw. Chemokinrezeptoren allgemein hervorragend als Ziel für eine zelldepletive Therapie geeignet. Von einer CCR5-Depletion wären im Gelenk somit fast alle Monozyten und T-Lymphozyten betroffen, während in der Blutbahn nur ein kleiner Teil dieser Zellen depletiert würde. Monozyten und T-Lymphozyten sind die dominierenden Zelltypen bei chronischen Gelenksentzündungen, wobei Monozyten insbesondere für die Gelenksdestruktion verantwortlich sind.

Überraschenderweise konnte jetzt gezeigt werden, daß mit Hilfe von neu entwickelten Antikörper konstrukten gezielt Chemokinrezeptor exprimierende, insbesondere CCR5 exprimierende, Leukozyten zerstört werden können.

Während gegenwärtige Behandlungsstrategien im wesentlichen eine symptomatische Therapie darstellen und den Verlauf der Gelenkserkrankung und die Gelenkdestruktion nur wenig beeinflussen können und z.B. Basistherapeutika ihre Wirkung erst nach mehreren Monaten entfalten und zudem wie die übrigen verwendeten Medikamente eine erhebliche Toxizität bei längerer Einnahme aufweisen, bewirken die erfindungsgemäßen Konstrukte innerhalb weniger Stunden im Gelenk eine fast vollständige Depletion von Chemokin-rezeptoren exprimierenden, insbesondere von CCR5[+] Monozyten/Makrophagen und T-Lymphozyten. Diese Zelltypen sind für die chronische Entzündung und Gelenksdestruktion verantwortlich. Aufgrund der Depletion kann eine langanhaltende Wirkung erzielt werden. Da Monozyten sehr effektiv eliminiert werden, ermöglicht der Einsatz des erfindungsgemäßen Antikörpers eine günstige Beeinflussung der Gelenksdestruktionen. Aufgrund der nicht-selektiven Hemmung des Immunsystems durch etablierte Basistherapeutika und TNFα-Blockade besteht. der Verdacht auf einev erhöhte Neigung zu Infekten und Tumorbildung. Aufgrund der Verteilung von Chemokinrezeptoren exprimierenden Zellen, insbesondere von CCR5 exprimierenden Leukozyten, können im Gegensatz dazu ganz überwiegend die am. Entzundungsprozeß beteiligten Leukozyten ausgeschaltet werden, ohne die Immunabwehr insgesamt wesentlich zu beeinftussen. Insgesamt ermöglicht der erfindungsgemäße Antikörper eine Therapie mit einem geringeren Nebenwirkungsspektrum, bei gleichzeitiger Verbesserung des Verlaufs der Gelenkserkrankung und Verhinderung von Gelenkdestruktionen.

Bei dem Konstrukt handelt es sich um einen bispezifischen Antikörper, der mit einer Spezifität, gegen den humanen Chemokinrezeptor CCR5, und mit der anderen Spezifität gegen ein CD3-Antigen auf der Oberfläche einer Effektorzelle, vorzugsweise auf der Oberfläche einer T-Zelle, gerichtet ist.

In einer besonders bevorzugten Ausführungsform handelt es sich bei dem bispezifischen Antikörper um einen single-chain-Antikörper

Die Erfindung umfaßt auch Antikörperkonstrukte, die einen Antikörper gegen einen CCR-5 Chemokinrezeptor umfassen, der über eine Multimerisierungsdomäne in Homo-/Heterodimere mit einem zweiten gegen CD3 gerichteten Antikörper in vitro und/oder in vivo verbunden werden können.

Allgemein handelt es sich bei den Antikörperkonstrukten, gemäß der Erfindung um Substanzen, die in der Lage sind, CCR5 exprimierende Zellen, insbesondere Leukozyten, zu zerstören, wobei diese selektive Zerstörung zur Behandlung von entzündlichen Erkrankungen (Autoimmunerkrankungen, allergische Erkrankungen u.ä.) eingesetzt wird.

Bispezifische Antikörper können wie unten beschrieben oder nach bekannten Methoden hergestellt werden; zu nennen sind hier beispielhaft chemisch gekoppelte Antikörper oder Antikörperfragmente, die Herstellung mittels der Hybridhybridon-Technik, die Herstellung als bispezifische single-chain-Antikörper, Diabodies und die Herstellung durch Zusammenlagerung von 2 Antikörpern über Multimerisierungsdomänen.

Bevorzugt handelt es sich bei den bispezifischen Antikörpern um single-chain-Antikörper. Dabei können die verschiedenen Teilbereiche dieser Antikörper mittels konventioneller Methoden zusammengefügt oder als "contiguous" Protein mittels rekombinanter DNA-Technologie hergestellt werden, z.B. derart, daß ein für eine chimäre oder humanisierte Antikörperkette kodierendes Nukleinsäuremolekül exprimiert wird, um eine contiguous Protein zu produzieren (siehe z.B. Mack et al. (1995) Proc. Natl. Acad. Sci. USA, Vol. 92, pp7021-7025).

In einer besonders bevorzugten Ausführangsform handelt es sich um einen single chain-Antikörper mit folgenden Fv-Fragmenten: sc-Fv-Fragment eines monoklonalen Antikörpers gegen den Chemokinrezeptor, vorzugsweise gegen CCR5, sowie ein sc-Fv-Fragment eines monoklonalen Antikörpers gegen CD3. Hierbei kann das Fv-Fragment gegen den Chemokinrezeptor als auch das Fv-Fragment gegen CD3 an N-terminaler Position stehen. Bevorzugt steht das Fv-Fragment gegen CCR5 an N-terminaler Position. Die Anordnung der VL und VH Antikörperdomänen kann in beiden Konstrukten variabel sein, bevorzugt besitzt das Fv-Fragment gegen CCR5 die Anordnung VL-VH und das Fv-Fragment gegen CD3 die Anordnung VH-VL. Die Verbindungslinker zwischen den variablen Domänen sowie zwischen beiden Fv-Fragmenten bestehen aus Peptidlinkern, bevorzugt aus einem hydrophilen flexiblen Glycin- und Serinhaltigen Linker aus 0-25 Aminosäuren. Eine zusätzliche Histidinkette aus 6xHis am C- oder N-terminaler Position kann zur leichteren Aufreinigung und Detektion verwendet werden.

Bispezifische sitigle-chain-Antikörper weisen im Vergleich zu konventionellen bispezifischen Antikörpern den Vorteil auf, daß sie nur aus einer Proteinkette bestehen und daher in ihrer Zusammensetzung genau definiert sind. Sie besitzen ein kleines Molekulargewicht von in der Regel <60 kD und lassen sich einfach und in großen Mengen rekombinant in geeigneten Zellinien, bspw in CHO-Zellen herstellen. Der wesentlichste Vorteil besteht allerdings darin, daß sie keine konstanten Antikörperdomänen besitzen und somit nur dann T-Lymphozyten zur Lyse aktivieren, wenn diese an ihre Zielzellen, also Chemokinrezeptoren exprimierende Zellen, gebunden sind. Single chain-Antikörper sind konventionellen bispezifischen Antikörper daher häufig deshalb überlegen, weil ihr klinischer Einsatz mit weniger bzw. geringeren Nebenwirkungen verbunden ist.

Die Erfindung betrifft weiter eine Zelle eukaryontischen oder prokaryontischen Ursprungs, die einen erfindüngsgemäßen bispezifischen Antikörper produziert.

Die Aufgabe der Erfindung wird desweiteren durch eine pharmazeutische Zusammensetzung, gelöst, umfassend mindestens einen bispezifischen Antikörper, in einer ausreichenden Menge, um Chemokinrezeptoren exprimierende Zellen zu zerstören, und ggf. einen pharmazeutisch annehmbaren Träger.

Pharmazeutische Zusammensetzungen umfassend den erfindungsgemäßen bispezifischen Antikörper sind für parenterale Verabreichung geeignet, d.h. subcutan, intramukulös, intravenös, intraartikulär, intraperitoneal, wobei die Zusammensetzungen für parenterale Verabreichung üblicherweise eine Lösung des. Antikörpers oder einen Cocktail davon, gelöst in einem annehmbaren Träger, vorzugsweise einen wäßrigen Träger, umfassen. Als wäßriger Träger eignen, sich beispielsweise Wasser, NaCl-Lösung und Glycin. Die Zusammensetzungen könner darüber hinaus übliche pharmazeutisch annehmbare. Hilfsstoffe enthalten, wie sie z.B. zur Einstellung eines bestimmten pH-Werts, als Puffersubstanz oder ähnlichern, eingesetzt werden.

Die Konzentration des bispezifischen Antikörpers in der Zusammensetzung kann sehr variieren, d.h. von weniger als etwa $10^{-7}$ Gew.-% bis 1 Gew.-%. Bevorzugt beträgt die Konzentration von $10^{-4}$ Gew.% bis $10^{-1}$ Gew.-%. Im Falle der intraartikulären Applikation sollte die Dosis des bispezifischen Antikörpers von 1 mg bis 0,00 mg und besonders bevorzugt ca. 0,02 mg des bispezifischen single chain-Antikörpers betragen.

So wäre z.B. für die intraartikuläre Applikation, z.B. in ein Kniegelenk, eine Zusammensetzung, umfassend etwa 20 μg des erfindungsgemäßen bispezifischen Antikörpers oder Chemokintoxins, gelöst in ca. 2-5 ml steriler Kochsalzlösung, z.B. gepuffert mit Phosphat oder Tris, geeignet. Ebenso wäre eine intravenöse Injektion als Applikationsform geeignet.

Eine weitere Aufgabe der Erfindung besteht darin, neue Verwendungsmöglichkeiten für den erfindungsgemäßen Antikörper aufzuzeigen.

Diese Aufgabe wird durch die Verwendung von mindestens einem erfindungsgemäßen Antikörper zur Depletion von Chemokinrezeptor exprimierenden Zellen gelöst. In einer bevorzugten Ausführungsform wird der erfindungsgemäße Antikörper zur Zubereitung einer pharmazeutischen Zusammensetzung für die Behandlung von chronischen Gelenksentzündungen und anderen Autoimmunkrankheiten oder von allergischen Erkrankungen eingesetzt.

Die selektive Chemokinrezeptor-Depletion, insbesondere die CCR5-Depletion hat im Vergleich zu bisherigen immunsuppressiven Therapien (wie z.B. Kortison, Methotrexat, Zytokinblockade) zwei wesentliche Vorteile: Erstens zielen bisherige Therapien nicht auf eine Depletion der infiltrierenden Leukozyten ab, sondern bewirken nur eine Unterdrückung der Aktivität. Dies führt dazu, daß es nach Absetzen der Behandlung rasch zu Rezidiven kommt und eine kontinuierliche Immunsuppression notwendig ist. Im Gegensatz dazu besteht bei einer Depletion der verantwortlichen Leukozyten im Gelenk die Hoffnung, daß bereits nach einer oder wenigen Behandlung eine langanhaltende Remission erzielt wird. Der zweite Vorteil einer Chemokinrezeptor-Depletion besteht darin, daß aufgrund der Verteilung von Chemokinrezeptor, insbesondere CCR5-, exprimierenden Zellen nur die am Entzündungsprozeß beteiligten Zellen ausgeschaltet werden und die übrigen Zellen weitgehend verschont bleiben.

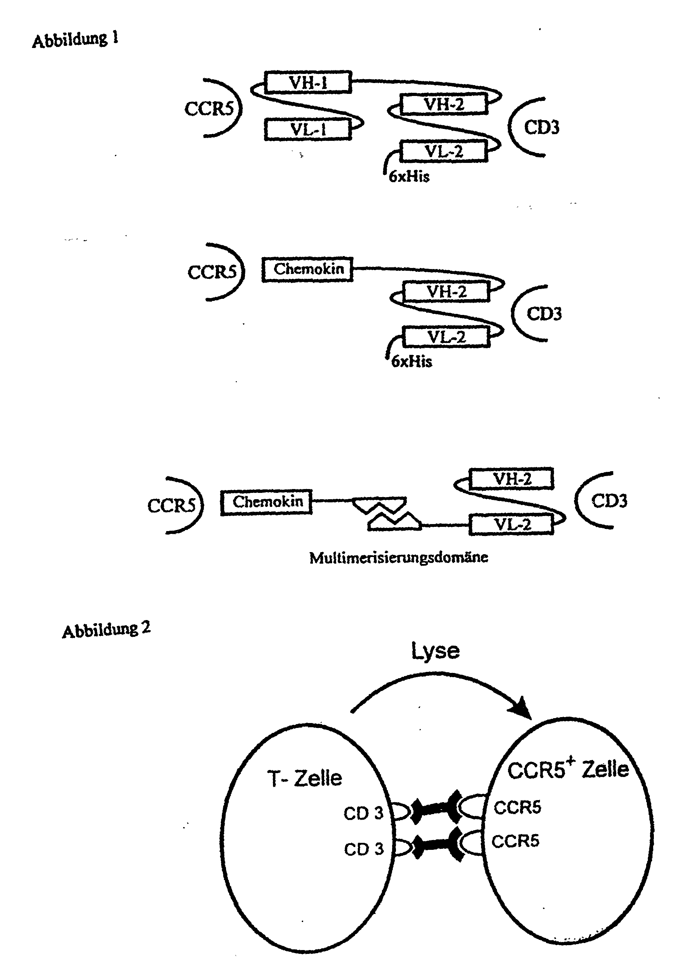

Der Wirkmechanismus des erfindungsgemäßen, bispezifischen Antikörpers, der mit einer Spezifität gegen einen Cheanokinrezeptor und mit der anderen Spezität gegen ein Antigen auf der Oberfläche von T-Zellen als Effektorzellen gerichtet ist, ist in Abbildung 1 (oben) und in Abbildung 2 anhand einer bevorzugten Ausführungsform der Erfindung verdeutlicht Wie in Abbildung 1 (oben) gezeigt, bindet ein bispezifischer Antikörper mit seinen beiden Armen an zwei verschiedenen Antigene. Dies sind der Chemokinrezeptor CCR5 und das Oberflächenmolekül CD3 auf T-Lymphozyten. Wie in Abbildung 2 dargestellt, führt der bispezifische Antikörper zu einer Vernetzung von T-Lymphozyten und CCR5. exprimierenden Zellen. Die Quervernetzung bewirkt daß T-Lymphozyten zur Lyse aktiviert werden und die mit ihnen verbundenen CCR5[+] Zellen zerstört werden. Eine Lyse von CCR5⁺ Zellen findet also nur statt, wenn auch T-Lymphozyten anwesend sind. Diese sind bei allen Formen der chronischen Gelenkentzündung in großer Zahl vorhanden. Auf diese Weise ist es insbesondere möglich, durch Injektion des bispezifischen Antikörpers in das entzündete Gelenk die für die Entzündung verantwortlichen Zellen gezielt zu eliminieren.

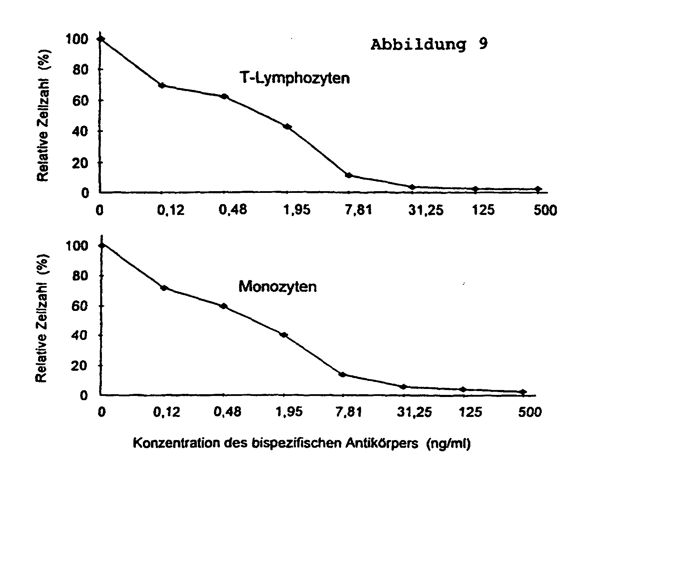

Weitere Ausführungsformen der Erfindung und ihre Wirkungsweise sind in den beigefügten Abbildungen 1 bis 9 veranschaulicht.

Aufgrund der Möglichkeit, Chemokinrezeptoren exprimierende Zellen gezielt zu depletieren, bietet sich das erfinderische Konzept nicht nur für chronische Gelenksentzündungen, sondern auch für rheumatische Erkrankungen und anderer Autoimmunerkrankungen an, bei denen eine ähnlich starke Anreicherung von Chemokinrezeptoren exprimierenden, insbesondere von CCR5 exprimierenden, Zellen gefunden wurde.

Während über mögliche Anwendungsgebiete von bispezifischen Antikörpern im Bereich der Tumortherapie bereits berichtet worden ist (z.B. in Kroesen, B. J. et al. (1993) Cancer Immunol. Immunother. 37:400-7; Nitta, T. et al. (1990) Lancet 335:368-71; Bolhuis, R.L. et al. (1992) Int J Cancer Suppl, 7:78-81; Canevari, S. et al. (1995) J. Natl. Cancer Inst. 87:1463-9; De Gast, G. et al. (1995) J. Hematother. 4:433-437; Kroesen, B. J. et al. (1994) Br. J. Cancer, 70:652-61; Tibben, J.G. et al. (1993) J. Natl. Cancer Inst. 85:1003-4; Mack, M et al (1995) P. N.A.S. USA 92,7021-7025), ist der Einsatz von bispezifischen Antikörpern zur Immunsuppression bislang noch nicht beschrieben worden.

Beschreibung der Abbildungen:

Abbildung 1 veranschaulicht erfindungsgemäße Antikörper- und nicht-erfindungsgemäße Chemokinkonstrukte, die eine Vernetzung von Targetzellen, hier CCR5 exprimierenden Zellen, und Effektorzelle, hier T-Zellen mit CD3 auf der Oberfläche, bewirken. Oben: bispezifischer Antikörper mit Spezifitäten sowohl gegen CCR5 als auch gegen CD3, Mitte: Chemokinkonstrukt bestehend aus einem Chemokin und einem Antikörper gegen CD3, Unten: Chemokin-konstrukt, bei dem ein Chemokin mit Multimerisierungsdomäne mit einem gegen CD3 gerichteten Antikörper über eine kompatible Multimerisierungsdomäne verbunden werden kann.

Die Quervemetzung führt, wie in Abbildung 2 schematisch dargestellt, daß T-Lymphozyten zur Lyse aktiviert werden und die mit ihnen vernetzten CCR5 positiven Zellen zerstört werden.

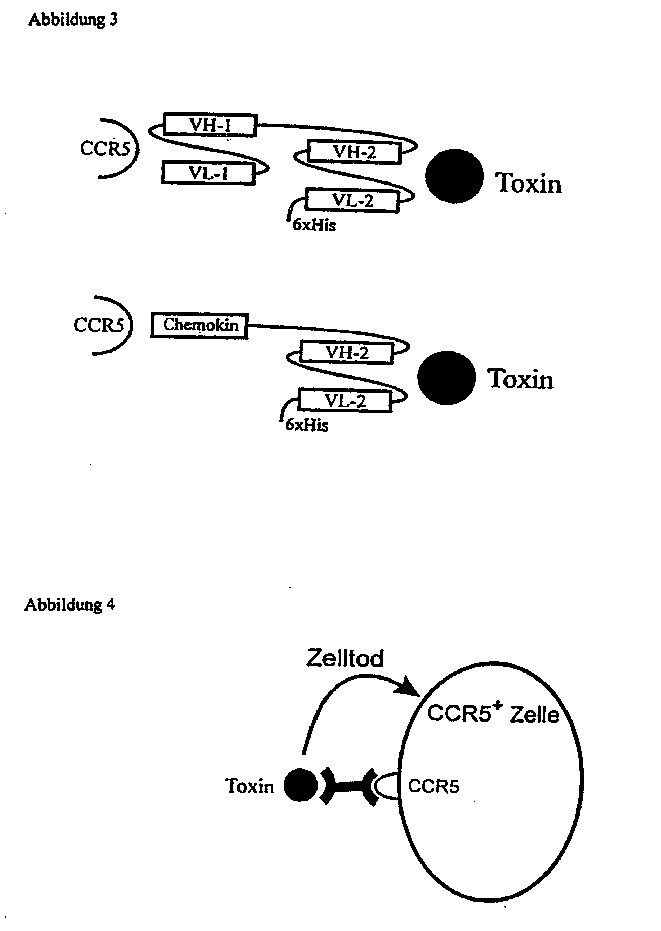

Abbildung 3 zeigt Antikörper- und Chemokinkenstnikte, die eine Verbindung zwischen Targetzellen, hier CCR5 exprimierenden Zellen, und einem Toxin herbeiführen. Oben: bispezifischer Antikörper mit Spezifitäten sowohl gegen CCR5 als auch gegen ein Toxin, Unten: Chemokinkonstrukt bestehend aus einem Chemokin und einem Antikörper gegen ein Toxin.

Die Vernetzung von Targetzelle und Toxin bewirkt, wie in Abbildung 4 veranschaulicht, den Zelltod der CCR5 positiven Targetzelle.

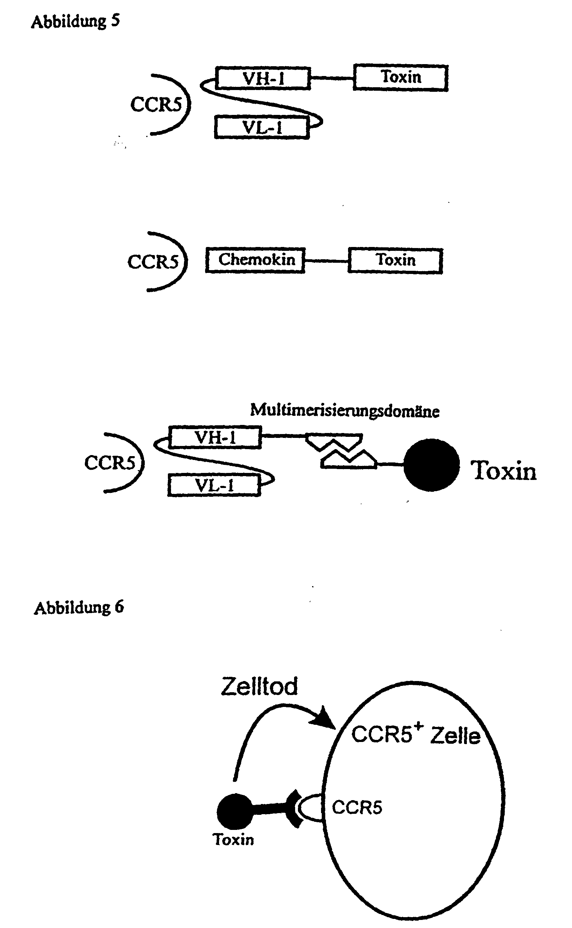

Abbildung 5 zeigt, ähnlich Abbildung 3, Antikörper- und Chemokinkonstrukte, die eine Verbindung zwischen einer Targetzelle und einem Toxin herbeiführen, wobei allerdings hier das Toxin nicht über die Spezifität eines gegen das Toxin gerichteten Antikörpers gebunden wird, sondern entweder kovalent mit einem gegen den Chemokinrezeptor, vorzugsweise gegen CCR5, gerichteten Antikörper (Abbildung 5, Oben) oder mit einem

Chemokin (Abbildung 5, Mitte) verbunden ist oder über eine Multimerisierungsdomäne mit einem gegen einen Chemokinrezeptor gerichteten Antikörper mit kompatibler Multimerisierungsdomäne verbindbar ist (Abbildung 5, Unten).

Auch in diesem Fall fuhrt, wie in Abbildung 6 skizziert, die Verbindung von Targetzelle und Toxin zum Zelltod der Targetzelle.

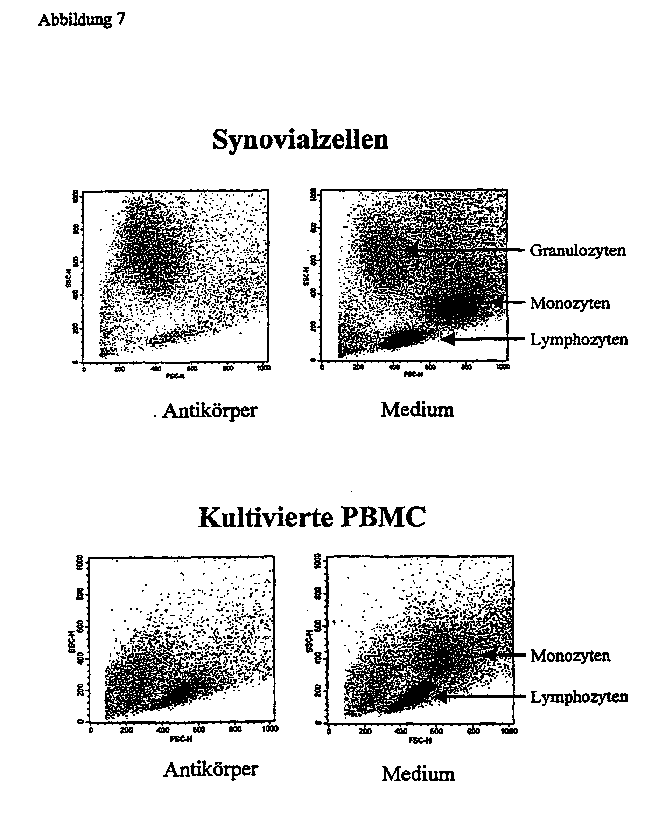

Abbildung 7 zeigt das Ergebnis einer FACS-Analyse (durchgeführt wie beschrieben in Mack et al. (1998) J. Exp. Med. 187, pp. 1215-1224), das die Depletion von CCR5 positiven T-Zellen und Monozyten aus der entzündeten synovialen Gelenkflüssigkeit von einem Patienten mit chronischer Polyarthritis bzw. von kultivierten peripheren Blutleukozyten (PBMC) verdeutlicht.

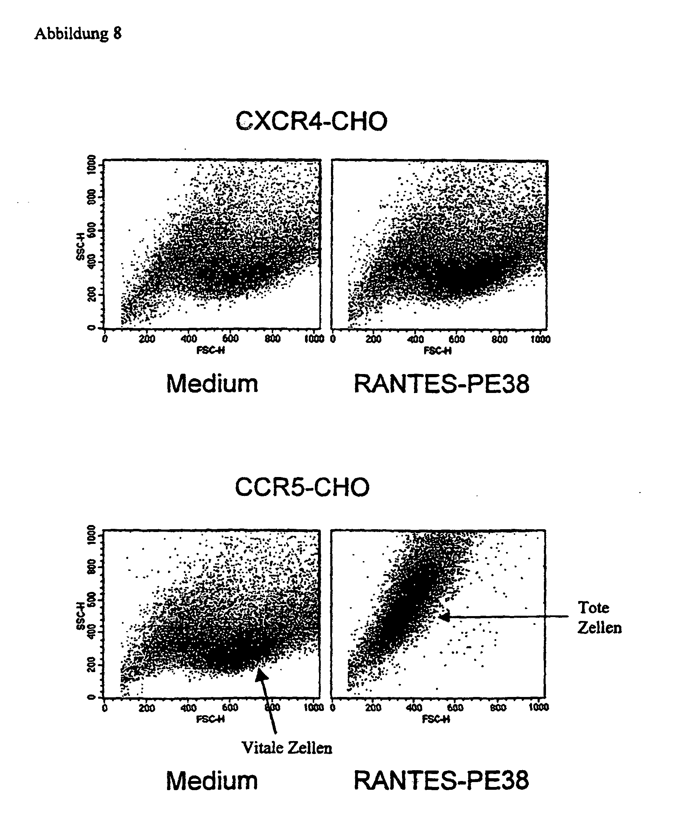

Abbildung 8 zeigt die Zytotoxizität des Chemokintoxins Ra-PE38, genauer gesagt die Zerstörung von CCR5 exprimierenden CHO-Zellen durch das Chemokintoxin Ra-PE38 (10 ng/ml) nach 24 Stunden Inkubation (unten rechts). CHO-Zellen, die statt CCR5 den Chemokinrezeptor CXCR4 exprimieren, werden durch Ra-PE38 nicht zerstört (oben), da RANTES nicht an CXCR4 bindet. Inkubation mit Medium bewirkt keine Zerstörung von CHO-Zellen. Ra-PE38 - Fusionsprotein aus RANTES und einem trunkierten Pseudomonasexotoxin mit einem Molekulargewicht von 38 kD.

Abbildung 8 veranschaulicht somit den in Abbildung 5, Mitte, skizzierten Ansatz.

Abbildung 9 gibt die Inkubation von Synovialflüssigkeit mit CD3-CCR5 bispezifischen Antikörpern über 48 Stunden wieder. Abbildung 9 ist somit im Zusammenhang mit Abbildung 7, oben, zu lesen. Es erfolgt eine konzentrationsabhängige Depletion der T-Lymphozyten und Monozyten. Bei einer Antikörperkonzentration von 125 ng/ml zeigte sich eine Reduktion der Monozyten und T-Lymphozyten um 96% bzw. 97,5%.

Die nachfolgenden Beispiele dienen der Veranschaulichung der Erfindung und stellen keine Einschränkung auf bestimmte Ausführungsformen bzw. Anwendungsbereiche dar. Vielmehr kann man sich eine Vielzahl weiterer Anwendungsmöglichkeiten der vorliegenden Erfindung vorstellen.

Beispiele

Beispiel 1: Herstellung eines bispezifischen Antikörpers

Für die Herstellung bispezifischer Antikörper kann bspw. die single-chain Technologie verwendet werden (Mack et al. (1995) Proc. Natl. Acad. Sci. U S A, 92:7021-7025; Mack et al. (1997) J. Immunol. 158: 3965-3970). Dazu werden, wie in den Abbildungen 1 (oben) und 3 (oben) schematisch dargestellt, die variablen Domänen der leichten (VL) und schweren (VH) Immunglobulin-kette von zwei verschiedenen Antikörpern in einer bestimmten Reihenfolge miteinander fusioniert und ggf. zusätzlich eine Histidinkette aus 6xHis angehängt. Die Fusion findet auf DNA-Ebene statt, so daß nach Expression eine Proteinkette mit vier verschiedenen variablen Domänen entsteht (vgl. Abbildungen 1 (oben) und 3 (oben)). Die angefügte Histidinkette ermöglicht eine einfache und effiziente Reinigung in einem Schritt über immobilisierte Ni-Ionen. Abbildung 1 (oben) zeigt eine bevorzugte Ausführungsform des bispezifischen Antikörpers, der an das CD3-Antigen auf der Oberfläche der Effektorzelle und an den humanen CCR5 auf der Oberfläche von Leukozyten als Targetzellen bindet.

Mittels RT-PCR werden die variablen Antikörperdomänen aus den Hybridomzellen, die den gewünschten Antikörper produzieren, amplifiziert. Die so gewonnenen PCR-Fragmente werden in einen Vektor kloniert und sequenziert. Anschließend wird mittels Fusions-PCR ein single-chain Antikörper für eine Spezifität generiert, indem zwischen den beiden variablen Antikörperdomänen ein Linker aus $(Gly_4Ser_1)_3$ eingefügt wird. In einer weiteren Fusions-PCR wird das Antikörperfragment gegen CCR5 mit dem bereits publizierten Antikörperfragment gegen CD3 fusioniert, wobei ein Linker bestehend aus $Gly_4Ser_1$ eingefugt wird (siehe Mack et al., supra). Die Anordnung der Domänen wird dabei folgendermaßen gewählt: VL(1)-VH(1)-VH(2)-VL(2), wobei (1) die Spezifität gegen CCR5 und (2) die Spezifität gegen CD3 bedeutet.

Beispiel 2: Expression und Aufreinigung des bispezifischen Antikörpers aus Beispiel 1

Zur Expression des bispezifischen Antikörpers wird die entsprechende DNA-Sequenz in einen eukaryontischen Expressionsvektor (z.B. PEF-DHFR, Mack et al. (1995) PNAS, supra) subkloniert und mittels Elektroporation in DHFR-defiziente CHO-Zellen transfiziert. Aus dem Überstand stabil transfizierter CHO-Zellen wird der bispezifische Antikörper mittles Affinitätschromatographie an Ni-NTA gereinigt, wobei die Elution mit pH-Erniedrigung stattfindet. Anschließend wird eine pH-Einstellung vorgenommen und das Protein auf eine geeignete Konzentration eingestellt.

Beispiel 3: In vitro-Tests an Blutleukozyten und an Leukozyten an entzündeten Gelenken

Für in vitro-Tests wird Gelenkergußflüssigkeit inclusive der enthaltenen Zellen mit dem bispezifischen Antikörper für ein oder mehrere Tage inkubiert. Bereits nach 24 Stunden ist ein fast vollständiges Verschwinden der CCR5 positiven Lymphozyten und Monozyten sichtbar. Nach längerer Inkubation findet bei der Mediumkontrolle eine Differenzierung der Monozyten in Makrophagen statt, die am Boden der Kulturflasche sichtbar sind. Bei entsprechender Inkubation mit dem bispezifischen Antikörper sind keine Makrophagen sichtbar.

Ein entsprechendes Ergebnis erhält man, wenn kultivierte PBMC mit dem bispezifischen Antikörper inkubiert werden. Hierbei tritt eine vollständige Depletion von CCR5 positiven Monozyten und eine weitgehende Depletion CCR5 positiver T-Lymphozyten ein. Die Depletion von CCR5 positiven T-Zellen und Monozyten in Abbildung 7 gezeigt.

Die Ergebnisse zeigen, daß das erfindungsgemäße Konstrukt gemäß Beispiel 1 in der Lage ist, CCR5 positive Monozyten vollständig zu zerstören. Dies gilt sowohl für Monozyten aus dem Gelenkpunktat als auch für Blutmonozyten, die bei ihrer Differenzierung zu Makrophagen CCR5 exprimieren. Die Depletion der Monozyten/Makrophagen findet innerhalb weniger Stunden (<24 h) statt. Besonders die Depletion der Monozyten/Makrophagen im Gelenk bietet einen wesentlichen therapeutischen Nutzen, da diese Zellen für die Gelenkdestruktion verantwortlich sind. Darüber hinaus ist auch für eine Aktivierung von T-Lymphozyten ein Zusammenspiel mit Makrophagen notwendig, so daß gleichzeitig eine Unterdrückung der T-Lymphozytenfunktion stattfindet.

Zusätzlich zur Depletion von Monozyten/Makrophagen konnte eine deutliche Reduktion in der Zahl der CCR5 positiven T-Lymphozyten beobachtet werden.

**Patentansprüche**

1. Bispezifischer Antikörper, der in der Lage ist an den humanen Chemokinrezeptor-5 (CCR5) auf der Oberfläche einer Targetzelle als erstes Antigen und an ein CD3-Antigen auf der Oberfläche einer Effektorzelle als zweites Antigen zu binden, wobei die Bindung des bispezifischen Antikörpers an den Chemokin rezeptor in der Zerstörung der Targetzelle resultiert.

2. Bispezifischer Antikörper nach Anspruch 1, der ein bispezifischer single-chain-Antikörper oder ein Diabody ist oder durch Zusammenlagerung von zwei Antikörpern. über Multimerisierungsdomänen in vitro und/oder in vivo hergestellt wird.

**3.** Bispezifischer Antikörper nach Anspruch 2, wobei der bispefiische single-chain-Antikörper ein scFv-Frägment eines monoklonalen Antikörpers gegen humanen Chemokinrezeptor-5 (CCR5) sowie ein scFv-Fragment eines monoklonalen Antikörpers gegen CD3 umfasst.

**4.** Bispezifischer Antikörper nach Anspruch 3, wobei das Fv-Fragment gegen CCR5 an der N-terminalen Position steht.

**5.** Bispezifischer Antikörper nach einem der Ansprüche 2. bis 4, wobei der bispezifische single-chain Antikörper die folgende Anordnung der Antikörperdomänen hat:

$$V_L(1)\text{-}V_H(1)\text{-}V_H(2)\text{-}V_L(2),$$

wobei (1) die Spezifität gegen CCR5 und (2) die Spezifität gegen CD3 bedeutet.

**6.** Bispezifischer Antikörper nach einem der Ansprüche 3 bis 5, wobei Verbindungslinker zwischen den variablen Domänen sowie zwischen beiden Fv-Fragmenten aus Peptidlinkern bestehen können.

**7.** Bispezifischer Antikörper nach, Anspruch 6, wobei die Verbindungslinker aus hydrophilen, flexiblen Glycin- und Serinhaltigen Linkern aus 0-25 Aminosäuren

**8.** Zelle eukaryontischen oder prokaryontischen Ursprungs, die einen bispezifischen Antikörper nach einem der vorangehenden Ansprüche produziert.

**9.** Pharmazeutische Zusammensetzung, umfassend mindestens einen bispezifischen Antikörper nach einem der Ansprüche 1 bis 7 in einer ausreichenden Menge, um Chemokinrezeptor exprimierende Zellen zu zerstören, und gegebenenfalls einen pharmazeutisch annehmbaren Träger.

**10.** Verwendung von mindestens einem bispezifischen Antikörper nach einem der Ansprüche 1 bis 7 zur Zubereitung einer pharmazeutischen Zusammensetzung für die Behandlung von chronischen Gelenksentzündungen und anderen Autoimmunkrankheiten und allergischen Erkrankungen.

**Claims**

**1.** A bispecific antibody capable of binding to the human chemokine receptor 5 (CCR5) on the surface of a target cell as a first antigen and to a CD3 antigen on the surface of an effector cell as a second antigen wherein the binding of the bispecific antibody to the chemokine receptor leads to the destruction of the target cell.

**2.** The bispecific antibody according to claim 1 which is a bispecific single-chain antibody or a diabody or which is produced in vitro and/or in vivo by connecting of two antibodies via multimerisation domains.

**3.** The bispecific antibody according to claim 2 wherein the bispecific single-chain antibody comprises an scFv fragment of a monoclonal antibody against human chemokine receptor 5 (CCR5) and an scFv fragment of a monoclonal antibody against CD3.

**4.** The bispecific antibody according to claim 3 wherein the Fv fragment against CCR5 is located in the N-terminal position.

**5.** The bispecific antibody according to any one of claims 2 to 4 wherein the antibody domains of the bispecific single-chain antibody are arranged as follows:

$$V_L(1)\text{-}V_H(1)\text{-}V_H(2)\text{-}V_L(2),$$

wherein (1) means the specificity against CCR5 and (2) means the specificity against CD3.

**6.** The bispecific antibody according to any one of claims 3 to 5 wherein linkers between the variable domains and between the two Fv fragments can consist of peptide linkers.

**7.** The bispecific antibody according to claim 6 wherein the linkers consist of hydrophilic, flexible glycin- and serine-containing linkers with 0 to 25 amino acids.

**8.** A cell of eukayotic or prokaryotic origin which produces a bispecific antibody according to any one of the preceding claims.

**9.** Pharmaceutical composition comprising at least one bispecific antibody according to any one of claims 1 to 7 in an amount sufficient to destroy chemokine receptor expressing cells, and optional a pharmaceutical acceptable carrier.

**10.** Use of at least one bispecific antibody according to any one of claims 1 to 7 for the preparation of a pharmaceutical composition for the treatment of chronic joint inflammations and other autoimmune diseases and allergic diseases.

## Revendications

1. Anticorps bispécifique, qui est en mesure de se fixer au récepteur humain de la chimiokine-5 (CCRS) à la surface d'une cellule-cible comme premier antigène et à un antigène CD3 à la surface d'une cellule effectrice comme deuxième antigène, la fixation de l'anticorps bispécifique au récepteur de la chimiokine se traduisant par la destruction de la cellule-cible.

2. Anticorps bispécifique selon la revendication 1, qui est un anticorps à chaîne unique bispécifique ou un diabody ou est produit in vitro et/ou in vivo par réunion de deux anticorps sur des domaines de multimérisation.

3. Anticorps bispécifique selon la revendication 2, dans lequel l'anticorps à chaîne unique bispécifique comporte un fragment scFv d'un anticorps monoclonal contre le récepteur humain de la chimiokine-5 (CCR5) ainsi qu'un fragment scFv d'un anticorps monoclonal contre le CD3.

4. Anticorps bispécifique selon la revendication 3, le fragment Fv contre le CCR5 se trouvant dans la position N-terminale.

5. Anticorps bispécifique selon l'une quelconque des revendications 2 à 4, l'anticorps à chaîne unique bispécifique ayant la disposition suivante des domaines d'anticorps :

$$V_L(1)\text{-}V_H(1)\text{-}V_H(2)\text{-}V_L(2).$$

(1) signifiant la spécificité vis-à-vis du CCR5 et (2), la spécificité vis-à-vis du CD3.

6. Anticorps bispécifique selon l'une quelconque des revendications 3 à 5, les segments de liaison entre les domaines variables ainsi qu'entre les deux fragments Fv pouvant être constitués de segments de liaison peptidiques.

7. Anticorps bispécifique selon la revendication 6, les segments de liaison étant constitués de segments de liaison flexibles, hydrophiles, constitués de 0-25 acides aminés contenant de la glycine et de la serine.

8. Cellule d'origine eucaryote ou procaryote qui produit un anticorps bispécifique selon l'une quelconque des revendications précédentes.

9. Composition pharmaceutique, contenant au moins un anticorps bispécifique selon l'une quelconque des revendications 1 à 7 en une quantité suffisante pour détruire les cellules exprimant le récepteur de chimiokine et, le cas échéant, un support acceptable sur le plan pharmaceutique.

10. Utilisation d'au moins un anticorps bispécifique selon l'une quelconque des revendications 1 à 7 pour la préparation d'une composition pharmaceutique pour le traitement d'inflammations articulaires chroniques et autres maladies auto-immunes et maladies allergiques.

Abbildung 1

Multimerisierungsdomäne

Abbildung 2

Lyse

T- Zelle

CCR5⁺ Zelle

CD 3

CD 3

CCR5

CCR5

**Abbildung 3**

**Abbildung 4**

Abbildung 5

Multimerisierungsdomäne

Abbildung 6

Abbildung 7

# Synovialzellen

. Antikörper      Medium

# Kultivierte PBMC

Antikörper      Medium

Abbildung 8

## CXCR4-CHO

Medium

RANTES-PE38

## CCR5-CHO

Tote Zellen

Vitale Zellen

Medium

RANTES-PE38

Abbildung 9